# EUROPEAN PATENT SPECIFICATION

(11) **EP 0 694 333 B1**
(45) Date of publication and mention of the grant of the patent: **29.09.1999**
(21) Application number: 94305595.4
(22) Date of filing: 28.07.1994
(51) Int. Cl.: B01J 31/04, C07C 51/215, C07C 51/31

(54) **Catalyst for preparing carboxylic acids**
Katalysator für die Herstellung von Carbonsäuren
Catalyseur pour la préparation d'acides carboxyliques

(43) Date of publication of application: 31.01.1996
(73) Proprietor: COUNCIL OF SCIENTIFIC & INDUSTRIAL RESEARCH, New Delhi 110001 (IN); ADARSH CHEMICALS & FERTILIZERS LTD, Gujarat 394210 (IN)
(72) Inventor: Kulsrestha, Gireindra Narain, Indian Institute of, Dehradun, U.P. (IN); Saxena, Mahendra Pratap, Indian Institute of, Dehradun, U.P. (IN); Gupta, Ashok Kumar, Laboratory of C.S.I.R. Dehradun (IN); Goyal, Hari Bhagwan, Indian Institute of, Dehradun, U.P. (IN); Prasad, Rameshwar, Indian Institute of, Dehradun, U.P. (IN); Rao, Turga Sundara Rama P., Indian Institute of, Dehradun, U.P. (IN); Patel, Prakash Dahyadbhai, Nariman Point, Bombay (IN)
(74) Representative: Skailes, Humphrey John

(56) References cited:
- FR-A- 1 549 026
- GB-A- 785 072
- US-A- 3 898 288
- JOURNAL OF ORGANIC CHEMISTRY, vol.38, no.5, 1973 pages 909 - 912 A. ONOPCHENKO ET AL. 'OXIDATION OF N-BUTANE WITH COBALT SALTS AND OXYGEN VIA ELECTRON TRANSFER'

## Description

This invention relates to a new catalyst which is useful for the preparation of carboxylic acids, a process for preparing the catalyst and its use in the production of carboxylic acids. This invention particularly relates to a catalyst which is useful for the preparation of carboxylic acids by oxidation of hydrocarbons. This invention more particularly relates to a catalyst which is highly efficient for oxidising specific hydrocarbons such as cyclopentane, cyclohexane, cycloheptane, C₄ -C₆ paraffins, toluene, 0-xylene, m-xylene, p-xylene and the like. The oxidation can be effected in one step in liquid phase by air or oxygen. The catalyst of the present invention is particularly useful for the preparation of dicarboxylic acids like adipic acid, pimelic acid, glutaric acid, phthalic acid etc. and mono carboxylic acids like acetic acid, benzoic acid.

Major applications of dicarboxylic acids are in the production of polyesters, polyamides, plasticizers, synthetic lubricants and alkyds. Adipic acid in particular finds wide application in the production of Nylon 66, special grade plasticizers, synthetic lubes, alkyds and polyurethanes. Monocarboxylic acids are used as solvents and in the synthesis of plasticizers and fine chemicals. Generally, dicarboxylic acids are produced by catalytic oxidation of hydrocarbons employing transition metal catalyst for example cobalt or manganese. In such processes, some times, the intermediates formed need to be separated and further subjected to oxidation depending on their reactivities with the oxidising agents employed.

As an example, adipic acid is made by oxidising cyclohexane to a mixture of cyclohexanone and cylohexanol in the first step followed by oxidation of this mixture by air or nitric acid to adipic acid in the second step. Currently, adipic acid is produced from cyclohexane by a two step oxidation process. In the first step cyclohexane is oxidised in a liquid phase at about 150°C by air or oxygen in presence of a cobalt or a manganese catalyst to form a mixture of cyclohexanone and cylohexanol. In the second step this mixture is oxidised by nitric acid at 50-80°C in presence of a vanadium-copper catalyst to adipic acid. The overall selectivity to adipic acid is around 70% based on cyclohexane. Other processes are also known which involve either high concentrations of hydroperoxides or utilise bromide-containing catalysts or high concentrations of cobalt salts as catalysts for oxidising cyclohexane.

The catalysts used for carrying out the above said processes have the following disadvantages:
1. Low (3-8%) conversion of cyclohexane per pass, requiring recycle of around 95% of the cyclohexane in the first step.
2. High concentration (0.2-10%) of hydroperoxides makes the process hazardous due to their explosive character at the reaction temperature employed viz. about 150°C.
3. The processes results in the formation of undesirable by product like lactones and oxy-acids during the reaction which are required to be separated. The separation of these undesired products requires complicated steps.
4. Use of bromide catalysts leads to severe corrosion of the apparatus. since the process operates at 5-25 kg/cm², such corrosion can also lead to explosion hazards. further the presence of gaseous pollutants containing oxides of nitrogen from nitric acid which is used as oxidising agent on the second step leads to severe corrosion and environmental problems.
5. Requirement of separate oxidation reactor systems for each oxidation step.

In order to raise the product selectivities, cobalt catalysts consisting of manganese, nickel, chromium, copper, or zinc salts along with cobalt salts have been used but these have not resulted in the removal of the drawbacks mentioned above.

Preparation of adipic acid by one step air oxidation of cyclohexane has been investigated earlier also. Publications of K. Tanaka in Chem. Tech. 4(9), 1974 p 555-559 in Chem Engg. news 52(15) 1974 p-24, in preprints (Div Petr Chem) Amer, Chem. Soc. 19(1), 1974 p 103-111, and Japanese Patent 75 19, 534 and of JGD schulz and A Onopchenko in J Org. Chem. 38, 1973 p. 3729-3733 and in J Org Chem 45, 1980 p 3716-3719 and US Patent 4,263,453 describe such single step processes using cobalt catalysts. A recent patent by Steinmetz et al US patent 4,902827 describes the use of Zirconium and Hafnium salts in fairly large concentrations along with cobalt. A US Patent 3,649,689, claims the air oxidation of cyclohexane to adipic acid using a catalyst comprising of cobalt carboxylates and bromine compounds.

In these previous attempts for preparing adipic acid by oxidation of cyclohexane in one step, the main focus is on the use of cobalt catalyst and at lower oxidation temperatures ranging from 80-130°C.

Tanaka also described the use of promoters like acetaldehyde, 2-butanone, cyclohexanone etc to convert in situ cobaltous to the catalytically active cobaltic state. These promoters ore to be continuously fed to the reactor to maintain the catalyst activity.

The drawbacks of the above said hitherto known one step processes for preparing adipic acid are:
1. The adipic acid formed is of low purity as initial high proportion of cobaltous salt leads to the formation of caprolactone that polymerises quickly to polycaprolactones.
2. Use of rare metals such as halfnium, zirconium etc. as cocatalysts is expensive and monitoring of their concentration is a complicated proposition.
3. Use of bromide as cocatalyst calls for special non-corrosive material of construction of the reactor and other process equipments thereby increasing the cost of the process.

The main objectives of the present invention are:
1. To provide a new catalyst which is useful for the oxidation of hydrocarbons such as cyclohexane, cyclopentane, cycloheptane, toluene, o-xylene, m-xylene and p-xylene and the like by air or oxygen to the corresponding carboxylic acids with product selectivity over 70% and higher (60-98%) hydrocarbon conversion.
2. To provide a process for the preparation of said novel catalyst from the corresponding mixtures of cobaltous and ferrous salts.
3. To provide an improved process for the production of carboxylic acids by oxidation of an appropriate hydrocarbon with air or oxygen using the novel catalyst consisting of a mixture of 70-99% by weight of cobaltic salt and 1-30% by weight of ferric salt with the high bydrocarbons conversion 60-98% and carboxylic acid selectivity over 70%. The acid component of the salt being acetate, propionate, naphthenate, adipate, phthalate or the like.
4. To provide an improved process for the preparation of dicarboxylic acids in which formation of undesired by-products such as lactones, oxy acids etc. are reduced substantially or eliminated.

Accordingly the present invention provides a novel catalyst useful for the preparation of carboxylic acids by the oxidation of hydrocarbons which comprises 70-99% by weight of cobalt salt and 1-30% by weight or ferric salt, the acid component of the salts being selected from acetate, propionate, naphthenate, adipate, phthalate or another anion of a C₁₋₆ alkane mono- or di-carboxylic acid or mono- or bi-cyclic aromatic mono- or di-carboxylic acid.

The present invention also provides a process for the preparation of the novel catalyst which is useful for the preparation of carboxylic acids by the oxidation of hydrocarbons, which comprises reacting a mixture of 70-99% by weight of the corresponding salts of cobaltous and 1-30% by weight of the corresponding salts of ferrous in the presence of an initiator, and a solvent selected from aliphatic mono carboxylic acids having 2-4 carbon atoms, or a mixture thereof and oxygen or air, at a temperature in the range of 60-150°C and pressure in the range of 1-50 kg/cm² for a period in the range of 0.25-8 hours.

The acid component of the cobaltous salt and the ferrous salt may be selected from acetate, propionate, naphthenate, benzoate, adipate, phthalate or the like. The amount of the cobaltous salt preferably ranges from 85-99% by weight and the ferrous salt from 1-15% by weight. The initiator used may be selected from 2-butanone, paraldehyde, cyclohexanone, cyclohexanol, acetaldehyde, partly oxidised hydrocarbons or their mixtures. The amount of initiator used may range from 10-100% by wt of the cobaltous salt. The solvent used may be selected from acetic acid, propionic acid, butyric acid and the like or their mixtures. The amount of initiator used preferably ranges from 30-50% by weight of the cobaltous salt. The solvent used may range from 2000-12000% by weight of the cobaltous salt.

The reaction may be effected at any oxygen or air space velocity, e.g. 10-90 h⁻¹ and preferably in the range of 30-60 h ⁻¹. The temperature used may preferably in the range of 80-130°C. The pressure employed may be preferably in the range of 1-10 kg/cm². The contacting time with air or oxygen may preferably be in the range of 0.25-3 hours.

According to another object of the present invention, there is provided an improved process for the preparation of carboxylic acids by oxidation of a hydrocarbon with oxygen or air which comprises :
(a) Contacting the hydrocarbon with a catalyst of the invention or produced by the process of the invention,
(b) Contacting the resulting mixture with oxygen or air at a pressure in the range of 1-70 kg/Cm² and a temperature range of 60-150°C for a period ranging between 1 and 8 hours at a space velocity of 1-200 h⁻¹,
(c) Separating the unreacted hydrocarbon and the catalyst by conventional methods,
(d) Separating the carboxylic acids formed by crystallisation or by fractional distillation, and
(e) Recycling, if desired, the resulted hydrocarbons, solvent and the catalyst from the steps (c) and (d) above.

In a preferred embodiment of the present invention, the catalyst to hydrocarbon ratio used in the reaction ranges between 0.01 to 0.5 by weight, the pressure used ranges from 10-50 kg/cm² and the temperature used ranges from 70-150°C and the space velocity ranges from 1-60 h⁻¹.

According to the specific feature of the present invention there is provided on improved process for the preparation of adipic acid which comprises :
(a) Contacting cyclohexane with the catalyst;
(b) Contacting the resulting mixture with oxygen or air at a pressure in the range of 1-70 Kg/cm² and a temperature in the range of 70-150°C for a period in the range of 1-8 hrs at a space velocity of 1-200 h⁻¹;
(c) Separating the unreacted cyclohexane and the catalyst by conventional methods;
(d) Separating the adipic acid formed by crystallisation or by fractional distillation; and
(e) recycling, if desired, the unreacted cyclohexane, the solvent and the catalyst from the steps (c) and (d).

The catalyst of the present invention when tested by oxidising hydrocarbons such as cyclohexane, cycloheptane, cyclopentane, toluene and xylenes, as explained above, was found to give selectivity of the production of carboxylic acids from 70 to 79% in cycloparaffin oxidation and 94-98% for toluene and xylenes with hydrocarbon conversions ranging between 80 and 90%. The product selectivities using the above described catalysts were found to be 2-4% higher than the maximum attainable using the hitherto known catalysts. The unreacted and partly oxidised hydrocarbons and the solvent after the separation of the acid products may be recycled to recover additional quantity of the acid, thereby increasing the efficiency of the process.

It is also to be noted that the examples given below are by way of illustrating the invention only and therefore should not be considered to limit the scope of the invention.

Examples 1-3 describe the process of preparing the novel catalyst of the present invention.

### Example I

A solution of 18 g cobaltous acetate, Co(OCOCH₃)₂.4H₂O, 2g ferrous acetate, 3 g cyclohexanone in 500 g acetic acid was heated to 95°C and treated with oxygen at a space velocity of 20 h⁻¹ for one hour till the combined concentration of cobaltic and ferric ions was about 85% of the total metal ions. At this stage the concentration (wt% of the total metal salts) of various ions in the catalyst so prepared was found to be : cobaltic acetate 79%, cobaltous acetate 11%, ferric acetate 6%, ferrous acetate 4%.

### Example II

A solution of 17 g cobaltous naphthenate, 3 g ferrous naphthenate and 3.5 g 2-butanone in 400 g acetic acid was heated to 100°C and treated with stirring with oxygen at a pressure of 20 kg/cm² for 2 hours. After this period about 89% of the combined ferrous and cobaltous ions are transformed to the higher oxidation state in the catalyst so prepared. The wt% of total ions, at this stage, of the catalyst prepared was found to be : cobaltic 79%, cobaltous 6%, ferric 13% and ferrous 2%.

### Example III

A solution of 16 g cobaltous adipate and 4 g ferrous adipate, 6 g paraldehyde, 150 g acetic acid was heated to 120°C and treated with oxygen at a pressure of 10 kg/cm² for 4 hours. After this period 90% of the combined ferrous and cobaltous ions were transformed to the higher oxidation state. The composition of the catalyst prepared based on wt% of total ions was found to be : cobaltic 75%, cobaltous 5%, ferric 15% and ferrous 5%.

The catalyst so prepared in the above said examples can be used as such for oxidation of hydrocarbons like cyclohexane, cyclopentane, cycloheptane, toluene, C₄-C₆ paraffins, o-xylene, m-xylene, p-xylene for the preparation of corresponding dicarboxylic or monocarboxylic acids such as adipic acid, glutaric acid, pimelic acid, benzoic acid, acetic acid, isophthalic acid, phthalic acid, terephthalic acid and the like.

### Example IV

165 g of the resulting catalyst mixture from example I above and 35 g cyclohexane were reacted with oxygen at a space velocity of 25 h⁻¹ at 95°C, 20 kg/cm² in a 500 ml autoclave.

At the end of three hours, the reaction mixture was flashed to collect 80 g of the material containing 8.7 g of unreacted cyclohexane, 10.2 g water and 6.1 g of acetic acid. The residue on coolling to 25°C yielded 24.0 g of crude 95% adipic acid crystals and 105 g filtrate plus acetic acid washings containing 9.6 g of adipic acid and 12.1 g partly oxidised products such as cyclohexanone, cyclohexanol, cyclohexyl acetate, monocyclohexyl adipate etc. and lower dicarboxylic acids which can be recycled. The cyclohexane conversion was 75.1% and the overall selectivity to adipic acid was 71%.

### Example V

35 g of cyclohexane, 165 g of the catalyst mixture from example II, was oxidised with oxygen at space velocity 20h⁻¹ at 110°C, 30 kg/cm². At the end of two hours, the reaction mixture was flashed to collect 100g of a material containing 10.5 g of cyclohexane, 10.1 g water and 79.4 acetic acid.

The residue on cooling and filtration yielded 25.3 g of crude 98.5% adipic acid. The filtrate contained 11.9 g adipic acid the cyclohexane conversion was 82% and overall selectivity to adipic acid was 73.8%.

### Example VI

A solution of 5.0 g cobaltous acetate tetrahydrate and 0.5 g of ferrous acetate 2 g cyclohexanone in 160 g glacial acetic acid was contacted at 95°C with oxygen at a rate of 100 ml per minute with stirring for 2 hours when around 90% cobaltous as well as ferrous salts were converted to cobaltic and ferric salts. 35 g of cyclohexane was than added and the mixture further reacted with oxygen at a space velocity of 25 h⁻¹ at 95°C and 49 Kg/cm² pressure. At the end of four hours the product was distilled to remove unreacted cyclohexane, water and some adipic acid. The residue was cooled and filtered to collect 23.8g of 98.5% adipic acid. The filtrate contained 9.1 g adipic acid. The cylcohexane conversion recorded was 71% and overall selectivity to adipic acid was 76.8%.

### Example VII

175g of the catalyst mixture from example III and 35 g cyclohexane was treated for 6 hours at 100°C at 30 Kg/cm² with oxygen at 50ml per minute at the outlet. Overall selectivity to adipic acid was 74.5% and conversion of cyclohexane was 80%.

Though the present invention is described with Examples given above with particular reference to the preparation of adipic acid it should not be construed that the invention is restricted to the preparation of adipic acid only. The process of the present invention can be used for the preparation of dicarboxylic acids such as glutaric acid, pimellic acid, phthalic acid, isophthalic acid and terephthalic acid and monocarboxylic acids such as acetic acids, propionic acid and benzoic acid. This has been exemplified in the Examples given below.

### Example VIII

A solution of 40g of p-xylene, 5.5g of the catalyst containing 80% by weight cobaltic acetate, 10% ferric acetate, 8% cobaltous acetate and 2% ferrous acetate was reacted with oxygen at space velocity 30 h⁻¹ at 130°C, 10 Kg/cm² for four hours. The oxidate was flashed to collect 30.0g of distillate containing, 4.0 g unreacted p-xylene, 8.2 g water and balance acetic acid. The residue was cooled and filtered, the solid was leached with acetic acid and dried to yield 51.9g of crude 98% terephthalic acid corresponding to 90% p-xylene conversion and 92% isophthalic acid selectivity. The solution when recycled in fresh run yielded 53.5g of terephthalic acid corresponding to 95% selectivity to terephthalic acid.

### Example IX

A solution of 40g of m-xylene, 6g of the catalyst containing (by weight) 85% cobaltic acetate, 8% ferric acetate 5% cobaltous acetate and 2% ferrous acetate and 200g acetic acid was reacted with oxygen at space velocity 25h⁻¹, at 135°C and 20 Kg/cm² for four hours. The oxidate was flashed to collect 18.5 g distillate consisting of 3.5g unreacted m-xylene, 8.5 g water and 6.5g acetic acid. The residue, after cooling to room temperature, was filtered and the solid leached with acetic acid and dried to yield 53.4g of 98% isophthalic acid, corresponding to 93.5% selectivity based on reacted m-xylene, which amounted to 91.2%. The filtrate when recycled in a fresh run resulted in 97% selectivity to isophthalic acid.

### Example X

A solution containing 30g cyclopentane, 5.0g catalyst consisting (by weight) 80% cobaltic acetate, 12% ferric acetate, 6% cobaltous acetate and 2% ferrous acetate and 150g acetic acid was reacted with air at space velocity 60 h⁻¹ at 95°C and 45Kg/cm² for four hours. The oxidate was flashed to collect 50 g distillate consisting of 4 g unreacted cyclopentane, 10g water and 36g acetic acid. The residue contained 34.3g glutaric acid corresponding to 86% cyclopentane conversion and 71% selectivity to glutaric acid.

### Example XI

200g solution in acetic acid and containing 4.0g catalyst consisting 85% cobaltic acetate 8% ferric acetate, 5% cobaltous acetate, and 2% ferrous acetic and 50 g toluene was reacted with oxygen at a space velocity of 40 h⁻¹ at 85°C for four hours. The oxidation product on and work up yielded 61.7g benzoic acid, 2.1g benzaldehyde and 1.0g unreacted toluene. This corresponded to 98% hydrocarbon conversion and 95% selectivity to benzoic acid and more than 40% benzaldehyde.

## Claims

1. A novel catalyst useful for the preparation of carboxylic acids by the oxidation of hydrocarbons which comprises 70-99% by wt. of cobaltic salt and 1-30% by wt. of ferric salt, the acid component of the salt being an anion of a C₁₋₆ alkane mono- or di-carboxylic acid or mono- or bi-cyclic aromatic mono- or di-carboxylic acid.

2. A novel catalyst as claimed in claim 1 wherein the salt is an acetate, propionate, naphthenate, adipate or phthalate.

3. A process for the preparation of a new catalyst useful for the preparation of carboxylic acids by the oxidation of hydrocarbons which comprises reacting a mixture of 70-99% by weight of a cobaltous salt of an acid as defined in claim 1 and 1-30% by weight of a ferrous salt of an acid as defined in claim 1, in the presence of an initiator, a solvent (selected from aliphatic monocarboxylic acids having 2-4 carbon atoms and mixtures thereof) and oxygen or air, at a temperature in the range of 60-150°C and pressure in the range of 1-50 kg/cm² for a period in the range of 0.25-8 hours.

4. A process as claimed in claim 3 wherein the reaction is effected at a space velocity in the range of 10-90 h⁻¹.

5. A process as claimed in claim 3 or claim 4 wherein the amount of cobaltous salt ranges from 80-99% by weight, ferrous salt ranges from 1-20% by weight, initiator ranges from 10-100% by weight of the cobaltous salt, the solvent ranges from 2000-12000% by weight of the cobaltous salt, the space velocity ranges from 30-60 h⁻¹, the temperature ranges from 80-150°C, the pressure ranges from 1-25 kg/cm² and the period ranges from 0.5-3 hours.

6. A process as claimed in any of claims 3 to 5 wherein the initiator is 2-butanone, cyclohexanol, cyclohexanone, acetaldehyde or a partly oxidised hydrocarbon.

7. A process as claimed in any of claims 3 to 6 wherein the solvent is acetic, propionic or butanoic acid or a mixture thereof.

8. A process for the preparation of carboxylic acid by oxidation of a hydrocarbon with oxygen or air which comprises:
(a) contacting the hydrocarbon with a catalyst as claimed in claim 1 or claim 2 or produced by a process as claimed in any of claims 3 to 7;
(b) contacting the resultant mixture with oxygen or air at a space velocity of 1-200 h⁻¹, a temperature in the range of 60-150°C, and a pressure in the range of 1-70 kg/cm² for a period in the range 1-8 hours;
(c) separating the unreacted hydrocarbon and the catalyst by conventional methods;
(d) separating the carboxylic acid formed by crystallisation or by fractional distillation; and
(e) recycling, if desired, the recovered hydrocarbons, solvent and the catalyst from the steps (c) and (d) above.

9. A process as claimed in claim 8 wherein the catalyst to hydrocarbon ratio used ranges between 0.01 to 0.5 by weight, the pressure used ranges from 10-50 kg/cm² and the temperature used ranges from 70-150°C and the space velocity ranges from 10-90 h⁻¹.

10. A process for the preparation of adipic acid by the oxidation of cyclohexane using a novel catalyst which comprises:
(a) contacting cyclohexane with a catalyst as claimed in claim 1 or claim 2 or produced by a process as claimed in any of claims 3 to 7;
(b) contacting the resulting mixture with air and oxygen at a pressure in the range of 1-70 kg/cm² and a temperature in the range of 70-150°C for a period in the range 1-8 hours at a space velocity of 1-200 h⁻¹;
(c) separating the unreacted cyclohexane and the catalyst by conventional methods;
(d) separating the adipic acid formed by crystallisation or by fractional distillation; and
(e) recycling, if desired, the unreacted cyclohexane, the solvent and the catalyst from the steps (c) and (d) above.

## Patentansprüche

1. Neuer Katalysator zur Herstellung von Carbonsäuren durch Oxidation von Kohlenwasserstoffen, gekennzeichnet durch 70 bis 99 Gew-% eines Kobalt(III)-salzes und 1 bis 30 Gew-% eines Eisen(III)-salzes, wobei die Säurekomponente des Salzes ein Anion einer C₁-C₆-Alkanmono- oder -dicarbonsäure oder einer mono- oder bicyclischen aromatischen Mono- oder Dicarbonsäure ist.

2. Neuer Katalysator nach Anspruch 1, worin das Salz ein Acetat, Propionat, Naphthenat, Adipat oder Phthalat ist.

3. Verfahren zur Herstellung eines neuen Katalysators zur Herstellung von Carbonsäuren durch Oxidation von Kohlenwasserstoffen, gekennzeichnet durch Umsetzung eines Gemisches von 70 bis 99 Gew-% eines Kobalt(II)-salzes einer Säure, wie in Anspruch 1 definiert, und 1 bis 30 Gew-% eines Eisen(II)-salzes einer Säure, wie in Anspruch 1 definiert, in Gegenwart eines Initiators, eines Lösungsmittels (ausgewählt unter aliphatischen Monocarbonsäuren mit 2 bis 4 Kohlenstoffatomen und Gemischen davon) und Sauerstoff oder Luft, bei einer Temperatur im Bereich von 60 bis 150 °C und einem Druck im Bereich von 1 bis 50 kg/cm² für einen Zeitraum im Bereich von 0,25 bis 8 Stunden.

4. Verfahren nach Anspruch 3, worin die Reaktion bei einer Raumgeschwindigkeit im Bereich von 10 bis 90 h⁻¹ bewirkt wird.

5. Verfahren nach Anspruch 3 oder 4, worin die Menge des Kobalt(II)-salzes im Bereich von 80 bis 99 Gew-% liegt, die des Eisen(II)-salzes im Bereich von 1 bis 20 Gew-% liegt, die des Inititators im Bereich von 10 bis 100 Gew-% bezogen auf das Kobalt(II)-salz liegt, die des Lösungsmittels im Bereich von 2000 bis 12000 Gew-% bezogen auf das Kobalt(II)-salz liegt, die Raumgeschwindigkeit liegt im Bereich von 30 bis 60 h⁻¹, die Temperatur liegt im Bereich von 80 bis 150 °C, der Druck liegt im Bereich von 1 bis 25 kg/cm², und die Zeit liegt im Bereich von 0,5 bis 3 Stunden.

6. Verfahren nach einem der Ansprüche 3 bis 5, worin der Initiator 2-Butanon, Cyclohexanol, Cyclohexanon, Acetaldehyd oder ein teilweise oxidierter Kohlenwasserstoff ist.

7. Verfahren nach einem der Ansprüche 3 bis 6, worin das Lösungsmittel Essig-, Propion- oder Butansäure oder ein Gemisch davon ist.

8. Verfahren zur Herstellung einer Carbonsäure durch Oxidation eines Kohlenwasserstoffs mit Sauerstoff oder Luft, gekennzeichnet durch
(a) In-Kontakt-bringen des Kohlenwasserstoffs mit einem Katalysator, wie in Anspruch 1 oder 2 beansprucht oder hergestellt nach einem der Ansprüche 3 bis 7;
(b) In-Kontakt-bringen des erhaltenen Gemisches mit Sauerstoff oder Luft bei einer Raumgeschwindigkeit von 1 bis 200 h⁻¹, einer Temperatur im Bereich von 60 bis 150 °C und einem Druck im Bereich von 1 bis 70 kg/cm² für einen Zeitraum im Bereich von 1 bis 8 Stunden;
(c) Abtrennung des nicht umgesetzten Kohlenwasserstoffs und des Katalysators nach üblichen Verfahren;
(d) Abtrennung der gebildeten Carbonsäure durch Kristallisation oder durch fraktionierte Destillation; und
(e) gewünschtenfalls Rückführung der zurückgewonnenen Kohlenwasserstoffe, des Lösungsmittels und des Katalysators aus den obigen Stufen (c) und (d).

9. Verfahren nach Anspruch 8, worin das Katalysator-zu-Kohlenwasserstoff-Verhältnis im Bereich zwischen 0,01 und 0,5 Gew-% liegt, der Druck liegt im Bereich von 10 bis 50 kg/cm², und die Temperatur liegt im Bereich von 70 bis 150 °C, und die Raumgeschwindigkeit liegt im Bereich von 10 bis 90 h⁻¹.

10. Verfahren zur Herstellung von Adipinsäure durch Oxidation von Cyclohexan unter Verwendung eines neuen Katalysators, gekennzeichnet durch
(a) In-Kontakt-bringen von Cyclohexan mit einem Katalysator, wie in Anspruch 1 oder 2 beansprucht oder hergestellt nach einem der Ansprüche 3 bis 7;
(b) In-Kontakt-bringen des erhaltenen Gemisches mit Luft und Sauerstoff bei einem Druck im Bereich von 1 bis 70 kg/cm² und einer Temperatur im Bereich von 70 bis 150 °C für einen Zeitraum im Bereich von 1 bis 8 Stunden bei einer Raumgeschwindigkeit von 1 bis 200 h⁻¹,;
(c) Abtrennung des nicht umgesetzten Cyclohexans und des Katalysators nach üblichen Verfahren;
(d) Abtrennung der gebildeten Adipinsäure durch Kristallisation oder durch fraktionierte Destillation; und
(e) gewünschtenfalls Rückführung des nicht umgesetzten Cyclohexans, des Lösungsmittels und des Katalysators aus den obigen Stufen (c) und (d).

## Revendications

1. Nouveau catalyseur pour la préparation d'acides carboxyliques par oxydation d'hydrocarbures, qui comprend de 70 à 99 % en masse de sel cobaltique et de 1 à 30 % en masse de sel ferrique, la composante acide du sel était l'anion correspondant à un acide mono- ou dicarboxylique en C₁₋₆ ou un acide mono- ou dicarboxylique aromatique mono- ou bicyclique.

2. Nouveau catalyseur selon la revendication 1, caractérisé en ce que le sel est un acétate, un propionate, un napthalénate, un adipate ou un phtalate.

3. Procédé de préparation d'un nouveau catalyseur, pour la préparation d'acides carboxyliques par oxydation d'hydrocarbures, qui comprend une étape consistant à faire réagir un mélange de 70 à 99 % en masse de sel cobalteux d'un acide répondant à la définition de la revendication 1 et de 1 à 30 % en masse de sel ferreux d'un acide répondant à la définition de la revendication 1, en présence d'un initiateur, d'un solvant (choisi parmi les acides monocarboxyliques aliphatiques comportant de 2 à 4 carbones et les mélanges de ces acides) et de l'oxygène ou de l'air, à une température de 60 à 150°C et à une pression de 1 à 50 kg/cm², pendant une durée de 0,25 à 8 heures.

4. Procédé selon la revendication 3 caractérisé en ce que la réaction est effectuée avec une vitesse spatiale horaire comprise entre 10 et 90 h⁻¹.

5. Procédé selon les revendications 3 ou 4 caractérisé en ce que la quantité de sel cobalteux est comprise entre 80 et 99 % en masse, la quantité de sel ferreux entre 1 et 20 %, en masse, la quantité d'initiateur entre 10 et 100 % en masse par rapport au sel cobalteux, la quantité de solvant entre 2 000 et 12 000 % en masse par rapport au sel cobalteux, la vitesse spatiale horaire entre 30 et 60 h⁻¹, la température entre 80 et 150°C, la pression entre 1 et 25 kg/cm² et le temps de réaction entre 0,5 et 3 heures.

6. Procédé selon l'une quelconque des revendications 3 à 5 caractérisé en ce que l'initiateur est la 2-butanone, le cyclohexanol, la cyclohexonone, l'acétaldéhyde ou un hydrocarbure partiellement oxydé.

7. Procédé selon l'une quelconque des revendications 3 à 6 caractérisé en ce que le solvant est l'acide acétique, propionique ou butanoïque ou un mélange de ces acides.

8. Procédé de préparation d'un acide carboxylique par oxydation d'un hydrocarbure par l'oxygène ou l'air qui comprend les étapes consistant à :
(a) mettre en contact l'hydrocarbure avec un catalyseur selon les revendications 1 ou 2 ou un catalyseur synthétisé à l'aide d'un procédé selon l'une quelconque des revendications 3 à 7 ;
(b) mettre en contact le mélange résultant avec de l'oxygène ou de l'air à une vitesse spatiale horaire de 1 à 200 h⁻¹, à une température de 60 à 150°C et à une pression de 1 à 70 kg/cm² pendant une durée de 1 à 8 heures ;
(c) séparer les hydrocarbures n'ayant pas réagi et le catalyseur par des méthodes conventionnelles ;
(d) séparer l'acide carboxylique formé par cristallisation ou distillation fractionnée, et
(e) recycler, si on le désire, les hydrocarbures, le solvant et le catalyseur issus des étapes (c) et (d) ci-dessus.

9. Procédé selon la revendication 8 caractérisé en ce que le rapport massique catalyseur/hydrocarbure est compris entre 0,01 et 0,5, la pression entre 10 et 50 kg/cm², la température entre 70 et 150°C et la vitesse spatiale horaire de 10 à 90 h⁻¹.

10. Procédé de préparation de l'acide adipique par oxydation du cyclohexane utilisant un nouveau catalyseur qui comprend les étapes consistant à :
(a) mettre en contact le cyclohexane avec un catalyseur selon les revendications 1 ou 2 ou un catalyseur synthétisé par un procédé selon l'une quelconque des revendications 3 à 7 ;
(b) mettre en contact le mélange résultant avec de l'air et de l'oxygène à une pression de 1 à 70 kg/cm² et à une température de 70 à 150°C pendant une durée de 1 à 8 heures à une vitesse spatiale horaire de 1 à 200 h⁻¹ ;
(c) séparer le cyclohexane n'ayant pas réagi et le catalyseur par des méthodes conventionnelles ;
(d) séparer l'acide adipique formé par cristallisation ou distillation fractionnée, et
(e) recycler, si on le désire, le cyclohexane n'ayant pas réagi, le solvant et le catalyseur issus des étapes (c) et (d) ci-dessus.
